# EUROPEAN PATENT APPLICATION

(11) **EP 4 306 539 A1**
(43) Date of publication of application: **17.01.2024**
(21) Application number: 22766092.5
(22) Date of filing: 21.01.2022
(51) Int. Cl.: C07K 16/08, A61K 39/25, A61P 31/22

(54) **ANTI-VARICELLA-ZOSTER VIRUS ANTIBODY AND USE THEREOF**

(30) Priority: 10.03.2021 CN 202110259834
(71) Applicant: Beijing Wisdomab Biotechnology Co., Ltd, Beijing 101111 (CN); Genrix(Shanghai) Biopharmaceutical Co., Ltd., Shanghai 201203 (CN); Chongqing Genrix Biopharmaceutial Co., Ltd., Chongqing 401319 (CN)
(72) Inventor: ZHANG, Xueping, Beijing (CN); LIU, Zhigang, Beijing (CN); ZHOU, Xiaowei, Beijing (CN); HAO, Xiaobo, Beijing (CN); HU, Junjie, Beijing (CN); LIU, Yulan, Beijing (CN)
(74) Representative: Nederlandsch Octrooibureau
(86) International application number: PCT/CN2022/073199
(87) International publication number: WO 2022/188565

(57) **Abstract**

Provided are an antibody against varicella-zoster virus, a nucleic acid molecule encoding the antibody, an expression vector comprising the nucleic acid molecule, a host cell comprising the nucleic acid molecule or the vector, a method for preparing and purifying the antibody, and the use of the antibody.

## Description

### CROSS REFERENCE TO RELATED APPLICATION

The present application claims priority to Chinese Patent Application No. 202110259834.4 filed on March 10, 2021, which is incorporated herein by reference in its entirety.

### TECHNICAL FIELD

The present application generally relates to the field of genetic engineering and antibody medicine. In particular, the present application relates to an anti-varicella-zoster virus antibody and use thereof. Developed in the present application is a novel anti-varicella-zoster virus antibody, and provided in the present application is a use of the antibody in the prevention or treatment of varicella and zoster.

### BACKGROUND

Varicella-zoster virus (VZV) belongs to the genus *Herpesvirus,* alpha subfamily *Herpesvirus,* and is an enveloped DNA virus. VZV is the smallest human herpes virus, having a genome length of approximately 125,000 bp, including more than 70 open reading frames, and encoding a variety of proteins (Davison, A. J., and J. E. Scott. 1986). There are at least six surface glycoproteins in VZV, such as gB (gp II), gC (gp IV), gE (gp I), gH (gp III), gI and gL. Glycoproteins gE, gB and gH are extremely abundant on the cell membranes of infected cells, and the antibodies induced by them are capable of neutralizing viruses (Davison, A. J., C. M. Edson. 1986). Glycoprotein gE is expressed in highest abundance in VZV-infected cells, is non-covalently linked to gI protein, and binds to the Fc segment of immunoglobulin IgG. The gB protein is the target of neutralizing antibodies and plays a role in viral invasion of the host cells. The amino acid sequence of gB protein is highly conserved in VZV and herpes simplex virus-1 (HSV-1) (Kapsenberk, J. G. 1964). The gH protein mediates the fusion between the viral envelope and the cell membrane as well as between cell membranes, facilitating virus spread among cells. Only coexpression of the gH protein with the gL protein allows the gH protein to be glycosylated and translocated to the cell surface (Forghani, B., L. Ni, and C. Grose. 1994).

VZV can infect human diploid cells and melanoma cells and can also replicate in VERO cells and primary African green monkey kidney cells. VZV enters cells by adsorbing heparin sulfate proteoglycans on the cell surface and then binding to a low -affinity second receptor, expresses viral proteins within the infected cells to make the cells form multinucleated giant cells (Zhu, Z., M. D. Gershon. 1995). Most viral particles are remained in the cytoplasmic vacuoles. Few free viruses are released to the outside of the cells.

VZV has only one serotype. Its genetic diversity is limited, and this genetic diversity does not affect its infectivity and virulence. VZV infection is highly species-specific. VAV has no animal reservoir hosts. Although it can infect some non-human primates and small animals, such as guinea pigs (Chen, J. 2003) and rats (Sadzot-Delvaux.1990), it does not cause diseases in these animals. Human is the only known natural host, and the skin is the main target organ of the virus. VZV can be transmitted via droplets and/or contact. First infection in children mainly causes varicella. Varicella is prevalent in temperate areas, and usually occurs in winter and spring. Lifelong immunity is generated after varicella infection, and cases of second infection are rare (Gershon, A. A. 1984). Residual VZV in the infected individuals can retrograde along the sensory nerve axons, or can be transferred to spinal dorsal root ganglia or cranial ganglia after fusing with neuron cells via infected T cells and lurks in the ganglia. When the body's immunity is lowered, the latent viruses are activated and replicated in large numbers, transferred to the skin through the sensory nerve axons, and causes herpes zoster as VZV-specific cellular immunity decreases. As herpes zoster arises from the reactivation of the latent viruses, so only patients with primary infection will suffer from herpes zoster, and the onset is not seasonal. Herpes zoster occurs mainly in people over 45 years old, and the incidence rate increases with age. The incidence rate of herpes zoster in the general population is (3-5)/1000 persons/year, with an annual increase of 2.5-5.0%. The incidence rate of herpes zoster in people over 75 years old can reach 10/1000 persons/year, and the incidence rate of herpes zoster in children is very low (Guess, H. A. 1985). Herpes zoster is more common in patients receiving immunosuppressive therapy and in patients with HIV infection. Herpes zoster is accompanied by severe pain in addition to skin lesions, and a common complication is Post Herpetic Neuralgia (PHN), which can last for months to years.

IgG, IgM and IgA antibodies are produced as a result of VZV infection. These antibodies bind to viral proteins including glycoproteins, regulatory proteins, structural proteins and enzymes (Bogger-Goren. 1984). The neutralizing antibodies mediate lysis of infected cells. Neutralizing antibodies that bind to the gE and gI proteins require complements for their neutralizing action, whereas antibodies that bind to the gB and gH/gL proteins do not require complements. IgG antibodies, especially antibodies against glycoproteins, exist in the body for a long time after first infection with varicella, protecting the body from second infection. Injection of immunoglobulins against VZV within 72 hours after exposure to VZV in neonates, unimmunized pregnant women, or immunocompromised individuals can inhibit infection and viral replication in the body (Zaia, J. A., 1983), reduce the severity of varicella and decrease the risk of varicella pneumonia, but injection of immunoglobulins in children with acute varicella cannot clinically ameliorate the course of the disease. Antiviral drugs can effectively reduce the severity of varicella and herpes zoster, shorten the disease course, and reduce the incidence of postherpetic neuralgia. At present, the treatment of herpes zoster is mainly non-specific antiviral therapy, with no specific antiviral drug.

Therefore, it is of great biological and medical significance to explore and develop anti-varicella-zoster virus antibodies due to clinical needs.

### SUMMARY OF THE INVENTION

In a first aspect, there is provided in the present application an antibody against varicella-zoster virus comprising a heavy chain variable region comprising the amino acid sequences of HCDR1, HCDR2 and HCDR3 and a light chain variable region comprising the amino acid sequences of LCDR1, LCDR2 and LCDR3, wherein
the amino acid sequence of HCDR1 is SGNYWN, the amino acid sequence of HCDR2 is YISYDGSTYYNPSLKN, the amino acid sequence of HCDR3 is GYYGYWFAY, the amino acid sequence of LCDR1 is RASSSVSYMH, the amino acid sequence of LCDR2 is ATSNLAS, and the amino acid sequence of LCDR3 is QQWSSNPFT; or
the amino acid sequence of HCDR1 is SGYYWN, the amino acid sequence of HCDR2 is YISYDGSNNYNTSLKN, the amino acid sequence of HCDR3 is EDVNYPPYALDY, the amino acid sequence of LCDR1 is RSSQSLVHSNGNTYLH, the amino acid sequence of LCDR2 is KVSNRFS, and the amino acid sequence of LCDR3 is SQSTHVPWT; or
the amino acid sequence of HCDR1 is SYTMS, the amino acid sequence of HCDR2 is FISNGGDNNYYADTVKG, the amino acid sequence of HCDR3 is HNGNWGFAY, the amino acid sequence of LCDR1 is SASSSISSNYLH, the amino acid sequence of LCDR2 is RTSNLAS, and the amino acid sequence of LCDR3 is QQGSSIPLT; or
the amino acid sequence of HCDR1 is TYAMH, the amino acid sequence of HCDR2 is VISYGGGNRYYAASVKG, the amino acid sequence of HCDR3 is ARDNHYFFGMDV, the amino acid sequence of LCDR1 is RASQGISSWLA, the amino acid sequence of LCDR2 is AASSLQS, and the amino acid sequence of LCDR3 is QQANSFPLT, QEGFYFPIN, QQATYFPIN or QQSSYFPIA;
wherein the amino acid sequences of HCDRs and LCDRs are defined according to Kabat.

In some embodiments of the first aspect, the amino acid sequence of the heavy chain variable region of the antibody is set forth in SEQ ID NO: 1, 3, 5, or 7.

In some embodiments of the first aspect, the amino acid sequence of the light chain variable region of the antibody is set forth in SEQ ID NO: 2, 4, 6, 8, 9, 10, or 28.

In some embodiments of the first aspect, the amino acid sequence of the heavy chain variable region of the antibody is set forth in SEQ ID NO:1, and the amino acid sequence of the light chain variable region of the antibody is set forth in SEQ ID NO:2; or
the amino acid sequence of the heavy chain variable region of the antibody is set forth in SEQ ID NO:3, and the amino acid sequence of the light chain variable region of the antibody is set forth in SEQ ID NO:4; or
the amino acid sequence of the heavy chain variable region of the antibody is set forth in SEQ ID NO:5, and the amino acid sequence of the light chain variable region of the antibody is set forth in SEQ ID NO:6;
the amino acid sequence of the heavy chain variable region of the antibody is set forth in SEQ ID NO:7, and the amino acid sequence of the light chain variable region of the antibody is set forth in SEQ ID NO:8; or
the amino acid sequence of the heavy chain variable region of the antibody is set forth in SEQ ID NO:7, and the amino acid sequence of the light chain variable region of the antibody is set forth in SEQ ID NO:9; or
the amino acid sequence of the heavy chain variable region of the antibody is set forth in SEQ ID NO:7, and the amino acid sequence of the light chain variable region of the antibody is set forth in SEQ ID NO: 10; or
the amino acid sequence of the heavy chain variable region of the antibody is set forth in SEQ ID NO:7, and the amino acid sequence of the light chain variable region of the antibody is set forth in SEQ ID NO:28.

In a second aspect, there is provided in the present application an antibody against varicella-zoster virus, wherein the amino acid sequence of the heavy chain variable region of the antibody has at least 90% identity to the amino acid sequence as set forth in any one of SEQ ID NOs: 1, 3, 5, and 7, and the amino acid sequence of the light chain variable region of the antibody has at least 90% identity to the amino acid sequence as set forth in any one of SEQ ID NOs: 2, 4, 6, 8, 9, 10, and 28.

In some embodiments of the first and second aspects, the antibody is a whole antibody, a Fab fragment, a F(ab')₂ fragment, or a single chain Fv (scFv).

In some embodiments of the first and second aspects, the antibody is a monoclonal antibody.

In some embodiments of the first and second aspects, the antibody further comprises a heavy chain constant region selected from an IgG1 subtype, an IgG2 subtype, or an IgG4 subtype.

In some embodiments of the first aspect and the second aspect, the antibody further comprises a light chain constant region selected from a kappa subtype or a lamda subtype.

In some embodiments of the first and second aspects, the antibody binds to the gH/gL protein of varicella-zoster virus; and/or the antibody mediates antibody-dependent cell-mediated cytotoxicity (ADCC).

In a third aspect, there is provided in the present application a nucleic acid molecule encoding the antibody of the first or second aspect.

In a fourth aspect, there is provided in the present application a pharmaceutical composition comprising the antibody of the first or second aspect and a pharmaceutically acceptable excipient, diluent or carrier.

In a fifth aspect, there is provided in the present application use of the antibody of the first or second aspect, the nucleic acid molecule of the third aspect, or the pharmaceutical composition of the fourth aspect in the manufacture of a medicament for the prevention or treatment of varicella and herpes zoster.

In a sixth aspect, there is provided in the present application a method of preventing or treating varicella and herpes zoster, comprising administering to a subject in need thereof the antibody of the first or second aspect, or the pharmaceutical composition of the fourth aspect.

### DESCRIPTION OF THE DRAWINGS

Figure 1 shows the results from an ELISA assay assessing the ability of the monoclonal antibodies against the varicella-zoster virus recombinant protein gH/gL to block the binding of purified anti-recombinant protein gH/gL phages to varicella-zoster virus recombinant protein gH/gL. Panels A-C show the blocking results of the monoclonal antibodies S8E1, S8B8 and S7A10against the varicella-zoster virus recombinant protein gH/gL, respectively.
Figure 2 shows the result from an ELISA assay assessing the ability of the monoclonal antibody H4H8-L1B7 against the varicella-zoster virus recombinant protein gH/gL to block the binding of purified anti-recombinant protein gH/gL phages to the recombinant protein gH/gL.
Figure 3 shows the results assessing the ADCC activities of the anti-varicella-zoster virus monoclonal antibodies using Jurkat-Dual-CD16a reporter cells.

### DESCRIPTION OF THE SEQUENCES

SEQ ID NO:1 shows the amino acid sequence of the heavy chain variable region of the monoclonal antibody S7A10.

SEQ ID NO:2 shows the amino acid sequence of the light chain variable region of the monoclonal antibody S7A10.

SEQ ID NO:3 shows the amino acid sequence of the heavy chain variable region of the monoclonal antibody S8E1.

SEQ ID NO:4 shows the amino acid sequence of the light chain variable region of the monoclonal antibody S8E1.

SEQ ID NO:5 shows the amino acid sequence of the heavy chain variable region of the monoclonal antibody S8B8.

SEQ ID NO:6 shows the amino acid sequence of the light chain variable region of the monoclonal antibody S8B8.

SEQ ID NO:7 shows the amino acid sequence of the heavy chain variable regions of the monoclonal antibodies H4H8-L1B7, H4H8-L14H8, H4H8-L13C1 and H4H8-L13C7.

SEQ ID NO:8 shows the amino acid sequence of the light chain variable region of the monoclonal antibody H4H8-L13C1.

SEQ ID NO:9 shows the amino acid sequence of the light chain variable region of the monoclonal antibody H4H8-L13C7.

SEQ ID NO: 10 shows the amino acid sequence of the light chain variable region of the monoclonal antibody H4H8-L14H8.

SEQ ID NO: 11 shows the amino acid sequence of the gH glycoprotein of varicella-zoster virus.

SEQ ID NO: 12 shows the amino acid sequence of the gL glycoprotein of varicella-zoster virus.

SEQ ID NO: 13 shows the amino acid sequence of the His tag.

SEQ ID NO: 14 shows the amino acid sequence of the heavy chain constant region of human *(homo sapiens)* IgG1 subtype.

SEQ ID NO: 15 shows the amino acid sequence of the heavy chain constant region of human *(homo sapiens)* IgG2 subtype.

SEQ ID NO: 16 shows the amino acid sequence of the heavy chain constant region of human *(homo sapiens)* IgG4 subtype.

SEQ ID NO: 17 shows the amino acid sequence of the heavy chain constant region of mouse *(mus musculus)* IgG1 subtype.

SEQ ID NO: 18 shows the amino acid sequence of the heavy chain constant region of mouse *(mus musculus)* IgG2a subtype.

SEQ ID NO: 19 shows the amino acid sequence of the light chain constant region of human *(homo sapiens)* kappa subtype.

SEQ ID NO:20 shows the amino acid sequence of the light chain constant region of human *(homo sapiens)* lamda subtype.

SEQ ID NO:21 shows the amino acid sequence of the light chain constant region of mouse *(mus musculus)* kappa subtype.

SEQ ID NO:22 shows the amino acid sequence of the light chain constant region of mouse *(mus musculus)* lamda subtype.

SEQ ID NO:23 shows the nucleotide sequence of the primer PmCGR.

SEQ ID NO:24 shows the nucleotide sequence of the primer PmCKR.

SEQ ID NO:25 shows the amino acid sequence of the single chain fragment variable S7A10.

SEQ ID NO:26 shows the amino acid sequence of the single chain fragment variable S8E1.

SEQ ID NO:27 shows the amino acid sequence of the single chain fragment variable S8B8.

SEQ ID NO:28 shows the amino acid sequence of the light chain variable region of the monoclonal antibody H4H8-L1B7.

### DETAILED DESCRIPTION OF THE INVENTION

The present inventors have obtained novel antibodies against varicella-zoster virus. In various aspects of the present application, there are provided novel antibodies against varicella-zoster virus, nucleic acid molecules encoding the antibodies or antigen binding fragments thereof, vectors comprising the nucleic acid molecules, host cells comprising the nucleic acid molecules or vectors, a method of preparing and purifying the antibodies, and the medical and biological use of the antibodies. Based on the sequences of the variable regions of the antibodies provided herein, the full-length antibody molecules can be constructed for use as drugs for preventing or treating varicella and herpes zoster.

Unless otherwise indicated, the inventions of the present application can be implemented using conventional molecular biology, microbiology, cell biology, biochemistry, and immunological techniques in the art.

Unless otherwise indicated, the terms used in the present application have the meanings commonly understood by those skilled in the art.

### DEFINITIONS

As used herein, the term "antibody" refers to an immunoglobulin molecule that is capable of specifically binding to a target via at least one antigen recognition site located in a variable region of the immunoglobulin molecule. Targets include, but are not limited to, carbohydrates, polynucleotides, lipids, and polypeptides, etc. As used herein, an "antibody" includes not only an intact (i.e., full-length) antibody, but also an antigen-binding fragment thereof (e.g., Fab, Fab', F(ab')₂, or Fv), a variant thereof, a fusion protein comprising portions of an antibody, a humanized antibody, a chimeric antibody, a diabody, a linear antibody, a single-chain fragment variable, a multi-specific antibody (e.g., a bispecific antibody), and any other modified configurations of an immunoglobulin molecule comprising a desired specific antigen recognition site, including a glycosylated variant of an antibody, an amino acid sequence variant of an antibody, and a covalently modified antibody.

Typically, an intact or full-length antibody comprises two heavy chains and two light chains. Each heavy chain contains a heavy chain variable region (VH) and first, second and third constant regions (CH1, CH2 and CH3). Each light chain contains a light chain variable region (VL) and a constant region (CL). A full-length antibody may be of any type of antibody, such as an IgD, IgE, IgG, IgA, or IgM (or their subtypes) antibody, but not necessarily belongs to any particular type. Immunoglobulins can be assigned to different types depending on their amino acid sequences of the heavy chain constant domains. Generally, immunoglobulins have five main types, i.e., IgA, IgD, IgE, IgG, and IgM, and some of these types can be further classified into subtypes (isotypes), such as IgG1, IgG2, IgG3, IgG4, IgA1, and IgA2. Heavy chain constant domains corresponding to individual immunoglobulin types are referred to as α, δ, ε, γ, and µ, respectively. Subunit structures and three-dimensional structures of different types of immunoglobulins are well known.

As used herein, the term "antigen binding fragment" or "antigen-binding portion" refers to a portion or region of an intact antibody molecule responsible for binding an antigen. An antigen binding domain can comprise a heavy chain variable region (VH), a light chain variable region (VL), or both. Each of VH and VL typically contains three complementarity determining regions, *i.e.,* CDR1, CDR2, and CDR3.

It is well known to those skilled in the art that complementarity determining regions (CDRs, usually including CDR1, CDR2 and CDR3) are the regions of a variable region that have mostly impact on the affinity and specificity of an antibody. The CDR amino acid sequences of VH or VL have two common definitions, *i.e.,* the Chothia definition and the Kabat definition (see, e.g., Kabat, "Sequences of Proteins of Immunological Interest", National Institutes of Health, Bethesda, Md. (1991); A1-Lazikani et al., J. Mol. Biol. 273: 927-948 (1997); and Martin et al., Proc. Natl. Acad. Sci. USA 86: 9268-9272 (1989)). For the amino acid sequences of the variable regions of a given antibody, the CDR amino acidsequences in VH and VL amino acid sequences can be determined according to the Chothia definition or the Kabat definition. In an embodiment of the present application, the CDR amino acid sequences are defined according to Kabat.

For the amino acid sequences of the variable regions of a given antibody, the CDR amino acid sequences in the amino acid sequences of variable regions can be determined in a variety of ways, for example, using online software Abysis (http://www.abysis.org/).

Examples of an antigen-binding fragment include, but are not limited to, (1) an Fab fragment, which can be a monovalent fragment having a VL-CL chain and a VH-CH1 chain; (2) an F(ab')₂ fragment, which can be a divalent fragment having two Fab' fragments linked by a disulfide bridge of the hinge region (*i.e.,* a dimer of Fab'); (3) an Fv fragment having VL and VH domains in a single arm of an antibody; (4) a single-chain Fv (scFv), which can be a single polypeptide chain consisting of a VH domain and a VL domain via a polypeptide linker; and (5) (scFv)₂, which can comprise two VH domains linked by a peptide linker and two VL domains that are combined with the two VH domains via a disulfide bridge.

As used herein, the term "specific binding" refers to a non-random binding reaction between two molecules, e.g., binding of an antibody to an antigen epitope.

As used herein, the term "monoclonal antibody" refers to an antibody from a substantially homogeneous antibody population, which means that the antibodies constituting the population are the same except for naturally occurring mutations which may occur in a small number of individual antibodies. Monoclonal antibodies described herein particularly include "chimeric" antibodies in which a portion of the heavy chain and/or the light chain is identical or homologous to a corresponding amino acid sequence in an antibody derived from a particular species or belonging to a particular antibody type or subtype, while the remaining portion of the heavy chain and/or the light chain is identical or homologous to a corresponding amino acid sequence in an antibody derived from another species or belonging to another antibody type or subtype; and also include fragments of such antibodies as long as they are capable of exhibiting the desired biological activity (see, U.S. Pat. No. 4,816,567; and Morrison et al., Proc. Natl. Acad. Sci. USA 81: 6851-6855 (1984)).

In a first aspect, there is provided in the present application an antibody against varicella-zoster virus comprising a heavy chain variable region comprising the amino acid sequences of HCDR1, HCDR2 and HCDR3 and a light chain variable region comprising the amino acid sequences of LCDR1, LCDR2 and LCDR3, wherein
the amino acid sequence of HCDR1 is SGNYWN, the amino acid sequence of HCDR2 is YISYDGSTYYNPSLKN, the amino acid sequence of HCDR3 is GYYGYWFAY, the amino acid sequence of LCDR1 is RASSSVSYMH, the amino acid sequence of LCDR2 is ATSNLAS, and the amino acid sequence of LCDR3 is QQWSSNPFT; or
the amino acid sequence of HCDR1 is SGYYWN, the amino acid sequence of HCDR2 is YISYDGSNNYNTSLKN, the amino acid sequence of HCDR3 is EDVNYPPYALDY, the amino acid sequence of LCDR1 is RSSQSLVHSNGNTYLH, the amino acid sequence of LCDR2 is KVSNRFS, and the amino acid sequence of LCDR3 is SQSTHVPWT; or
the amino acid sequence of HCDR1 is SYTMS, the amino acid sequence of HCDR2 is FISNGGDNNYYADTVKG, the amino acid sequence of HCDR3 is HNGNWGFAY, the amino acid sequence of LCDR1 is SASSSISSNYLH, the amino acid sequence of LCDR2 is RTSNLAS, and the amino acid sequence of LCDR3 is QQGSSIPLT; or
the amino acid sequence of HCDR1 is TYAMH, the amino acid sequence of HCDR2 is VISYGGGNRYYAASVKG, the amino acid sequence of HCDR3 is ARDNHYFFGMDV, the amino acid sequence of LCDR1 is RASQGISSWLA, the amino acid sequence of LCDR2 is AASSLQS, and the amino acid sequence of LCDR3 is QQANSFPLT, QEGFYFPIN, QQATYFPIN or QQSSYFPIA;
wherein the amino acid sequences of HCDRs and LCDRs are defined according to Kabat.

In some embodiments of the first aspect, the amino acid sequence of the heavy chain variable region of the antibody is set forth in SEQ ID NO: 1, 3, 5, or 7.

In some embodiments of the first aspect, the amino acid sequence of the light chain variable region of the antibody is set forth in SEQ ID NO: 2, 4, 6, 8, 9, 10, or 28.

In some embodiments of the first aspect, the amino acid sequence of the heavy chain variable region of the antibody is set forth in SEQ ID NO:1, and the amino acid sequence of the light chain variable region of the antibody is set forth in SEQ ID NO:2; or
the amino acid sequence of the heavy chain variable region of the antibody is set forth in SEQ ID NO:3, and the amino acid sequence of the light chain variable region of the antibody is set forth in SEQ ID NO:4; or
the amino acid sequence of the heavy chain variable region of the antibody is set forth in SEQ ID NO:5, and the amino acid sequence of the light chain variable region of the antibody is set forth in SEQ ID NO:6;
the amino acid sequence of the heavy chain variable region of the antibody is set forth in SEQ ID NO:7, and the amino acid sequence of the light chain variable region of the antibody is set forth in SEQ ID NO:8; or
the amino acid sequence of the heavy chain variable region of the antibody is set forth in SEQ ID NO:7, and the amino acid sequence of the light chain variable region of the antibody is set forth in SEQ ID NO:9; or
the amino acid sequence of the heavy chain variable region of the antibody is set forth in SEQ ID NO:7, and the amino acid sequence of the light chain variable region of the antibody is set forth in SEQ ID NO: 10; or
the amino acid sequence of the heavy chain variable region of the antibody is set forth in SEQ ID NO:7, and the amino acid sequence of the light chain variable region of the antibody is set forth in SEQ ID NO:28.

In a second aspect, there is provided in the present application an antibody against varicella-zoster virus, wherein the amino acid sequence of the heavy chain variable region of the antibody has at least 90% identity to the amino acid sequence as set forth in any one of SEQ ID NOs: 1, 3, 5, and 7, and the amino acid sequence of the light chain variable region of the antibody has at least 90% identity to the amino acid sequence as set forth in any one of SEQ ID NOs: 2, 4, 6, 8, 9, 10, and 28.

In some embodiments of the second aspect, the amino acid sequence of the heavy chain variable region of the antibody has at least 90%, 91%, 92%, 93%, 94%, 95%, 96%, 97%, 98%, 99% or more homology to the amino acid sequence as set forth in any one of SEQ ID NOs: 1, 3, 5, and 7.

In some embodiments of the second aspect, the amino acid sequence of the light chain variable region of the antibody has at least 90%, 91%, 92%, 93%, 94%, 95%, 96%, 97%, 98%, 99%, or more homology to the amino acid sequence as set forth in any one of SEQ ID NOs: 2, 4, 6, 8, 9, 10, and 28.

In some embodiments of the second aspect, the amino acid sequence of the heavy chain variable region of the antibody differs from the amino acid sequence as set forth in any one of SEQ ID NOs: 1, 3, 5, and 7 by about 1, 2, 3, 4, 5, 6, 7, 8, 9, or 10 amino acid substitutions, deletions, and/or additions.

In some embodiments of the second aspect, the amino acid sequence of the light chain variable region of the antibody differs from the amino acid sequence as set forth in any one of SEQ ID NOs: 2, 4, 6, 8, 9, 10, and 28 by about 1, 2, 3, 4, 5, 6, 7, 8, 9, or 10 amino acid substitutions, deletions, and/or additions.

In some embodiments of the second aspect, the C-terminal or N-terminal region of the amino acid sequence as set forth in any one of SEQ ID NOs: 1, 3, 5, and 7 can also be truncated by about 1, 2, 3, 4, 5, 6, 7, 8, 9, 10, 11, 12, 13, 14, 15, 20, 25 or more amino acids while still retaining the function similar to that of the heavy chain variable region of the antibody.

In some embodiments of the second aspect, 1, 2, 3, 4, 5, 6, 7, 8, 9, 10, 11, 12, 13, 14, 15, 20, 25 or more amino acids can also be added to the C-terminal or N-terminal region of the amino acid sequence as set forth in any one of SEQ ID NOs: 1, 3, 5, and 7, and the resulting amino acid sequences still retain the function similar to that of the heavy chain variable region of the antibody.

In some embodiments of the second aspect, 1, 2, 3, 4, 5, 6, 7, 8, 9, 10, 11, 12, 13, 14, 15, 20, 25 or more amino acids can also be added or deleted at a region other than the C-terminus or the N-terminus of the amino acid sequence as set forth in any one of SEQ ID NOs: 1, 3, 5, and 7, provided that the altered amino acid sequences substantially retain the function similar to that of the heavy chain variable region of the antibody.

In some embodiments of the second aspect, the C-terminal or N-terminal region of the amino acid sequence as set forth in any one of SEQ ID NOs: 2, 4, 6, 8, 9, 10, and 28 can also be truncated by about 1, 2, 3, 4, 5, 6, 7, 8, 9, 10, 11, 12, 13, 14, 15, 20, 25 or more amino acids while still retaining the function similar to that of the light chain variable region of the antibody.

In some embodiments of the second aspect, 1, 2, 3, 4, 5, 6, 7, 8, 9, 10, 11, 12, 13, 14, 15, 20, 25 or more amino acids can also be added to the C-terminal or N-terminal region of the amino acid sequence as set forth in any one of SEQ ID NOs: 2, 4, 6, 8, 9, 10, and 28, and the resulting amino acid sequences still retain the function similar to that of the light chain variable region of the antibody.

In some embodiments of the second aspect, 1, 2, 3, 4, 5, 6, 7, 8, 9, 10, 11, 12, 13, 14, 15, 20, 25 or more amino acids can also be added or deleted at a region other than the C-terminus or the N-terminus of the amino acid sequence as set forth in any one of SEQ ID NOs: 2, 4, 6, 8, 9, 10, and 28, provided that the altered amino acid sequences substantially retain the function similar to that of the light chain variable region of the antibody.

In some embodiments of the first and second aspects, the antibody is a whole antibody, a Fab fragment, a F(ab')₂ fragment, or a single chain Fv (scFv).

In some embodiments of the first and second aspects, the antibody is a whole human antibody.

In some embodiments of the first and second aspects, the antibody is a monoclonal antibody.

In some embodiments of the first and second aspects, the antibody further comprises a heavy chain constant region selected from an IgG1 subtype, an IgG2 subtype, or an IgG4 subtype.

In some specific embodiments of the first and second aspects, the heavy chain constant region is an IgG1 subtype.

In some embodiments of the first aspect and the second aspect, the antibody further comprises a light chain constant region selected from a kappa subtype or a lamda subtype.

In some specific embodiments of the first aspect and the second aspect, the light chain constant region is a kappa subtype.

In some embodiments of the first and second aspects, the antibody binds to the gH/gL protein of varicella-zoster virus; and/or the antibody mediates antibody-dependent cell-mediated cytotoxicity (ADCC).

In a third aspect, there is provided in the present application a nucleic acid molecule encoding the antibody of the first or second aspect.

In some embodiments, the nucleic acid molecule is operably linked to a regulatory amino acid sequence that can be recognized by a host cell transformed with the vector.

In a fourth aspect, there is provided in the present application a pharmaceutical composition comprising the antibody of the first or second aspect, and a pharmaceutically acceptable excipient, diluent, or carrier.

In some embodiments, the pharmaceutical composition is used for preventing or treating varicella and herpes zoster.

In some embodiments, the pharmaceutical composition can further comprise one or more of a lubricant, such as talc, magnesium stearate, and mineral oil; a wetting agent; an emulsifier; a suspending agent; a preservative such as benzoic acid, sorbic acid and calcium propionate; a sweetening agent and/or a flavoring agent.

In some embodiments, the pharmaceutical composition herein can be formulated as a tablet, a pill, a powder, a lozenge, an elixir, a suspension, an emulsion, a solution, a syrup, a suppository, or a capsule.

In some embodiments, the pharmaceutical composition of the present application can be delivered using any physiologically acceptable administration route including, but not limited to, oral administration, parenteral administration, nasal administration, rectal administration, intraperitoneal administration, intravascular injection, subcutaneous administration, transdermal administration, or inhalation administration.

In some embodiments, a pharmaceutical composition for therapeutic use can be formulated for storage in the form of a lyophilized formulation or an aqueous solution by mixing an agent with desired purity with a pharmaceutically acceptable carrier or excipient where appropriate.

In a fifth aspect, there is provided in the present application use of the antibody of the first or second aspect, the nucleic acid molecule of the third aspect, or the pharmaceutical composition of the fourth aspect in the manufacture of a medicament for the prevention or treatment of varicella and herpes zoster.

In a sixth aspect, there is provided in the present application a method of preventing or treating varicella and herpes zoster, comprising administering to a subject in need thereof the antibody of the first or second aspect, or the pharmaceutical composition of the fourth aspect.

In other aspects, there is also provided in the present application an isolated nucleic acid molecule encoding the antibody or the light or heavy chain thereof of the present invention, as well as a vector comprising the nucleic acid molecule, a host cell comprising the vector, and a method of producing the antibody. In some embodiments, the nucleic acid molecule is operably linked to a regulatory amino acid sequence that can be recognized by a host cell transformed with the vector. In some embodiments, the method of producing the antibody includes culturing the host cell to facilitate expression of the nucleic acid. In some embodiments, the method of producing the antibody further comprises recovering the antibody from the host cell culture medium.

In addition, the antibody specific for varicella-zoster virus described herein can also be used to detect the presence of varicella-zoster virus in a biological sample. Antibody-based detection methods are well known in the art and include, for example, ELISA, immunoblot, radioimmunoassay, immunofluorescence, immunoprecipitation, and other related techniques.

It is to be understood that the foregoing detailed description is intended only to enable those skilled in the art to have better understanding of the present application and is not intended to cause limitations in any way. Various modifications and variations can be made to the described embodiments by those skilled in the art.

### Examples

The following Examples are for purposes of illustration only and are not intended to limit the scope of the present application.

### Example 1: Preparation of the recombinant proteins

The gH glycoprotein (SEQ ID NO: 11) and gL glycoprotein (SEQ ID NO: 12) are needed in the preparation and identification of an antibody specific for varicella-zoster virus. gH and gL can only be efficiently secreted and expressed after they are separately synthesized in cells and undergo folding together to form a gH/gL dimer. There are a large number of post-translational modifications (e.g., glycosylation or disulfide bonds, etc) in the gH/gL dimer. Therefore, the use of the mammal cell expression system would be more favorable for maintaining the structure and function of the recombinant proteins. Meanwhile, the addition of the His tag (His, SEQ ID NO: 13) to the C-terminus of the gH glycoprotein will be more favorable for the purification of the recombinant proteins and the identification of the monoclonal antibody functions. Genes encoding the gH recombinant protein (comprising His tags) and the gL recombinant protein were designed and synthesized according to the amino acid sequences of the gH and gL glycoproteins of the varicella-zoster virus strain Oka from the Uniprot database. The synthetic genes encoding the gH-His and gL recombinant proteins were cloned into suitable eukaryotic expression vectors (such as pcDNA3.1, Invitrogen Inc., etc) using conventional molecular biology techniques, respectively, and the prepared recombinant protein expression plasmids were then transfected into HEK293 cells (such as HEK293F, Invitrogen Inc.) using liposomes (such as 293fectin, Invitrogen Inc., etc) or other transfection reagents (such as PEI, etc). The cells were cultured in suspension in serum-free media for 3-5 days. The culture supernatant was then harvested by centrifugation. The recombinant protein in the supernatant was subjected to one-step purification using a metal chelate affinity chromatography column (such as HisTrap FF, GE Inc., etc). The recombinant protein preservation buffer was then replaced with PBS (pH 7.0) or other suitable buffers using a desalting column (such as Hitrap desaulting, GE Inc., etc). If necessary, the sample can be sterilized by filtration and then stored in aliquots at -20°C.

In the preparation of the recombinant antibody, the heavy chain constant region of the antibody can be a human IgG1 subtype (SEQ ID NO:14), a human IgG2 subtype (SEQ ID NO:15), a human IgG4 subtype (SEQ ID NO:16), or a mouse IgG1 subtype (SEQ ID NO:17), a mouse IgG2a (SEQ ID NO:18) subtype, and the light chain constant region can be a human κ subtype (SEQ ID NO:19), a human λ subtype (SEQ ID NO:20), a mouse κ subtype (SEQ ID NO:21), or a mouse λ subtype (SEQ ID NO: 22). The nucleotide sequences encoding the heavy chain variable region and the light chain variable region of the antibody were cloned into eukaryotic expression vectors (such as pcDNA3.1, Invitrogen Inc.) fused with the nucleotide sequences encoding the heavy chain constant region and the light chain constant region, respectively, by conventional molecular biological means, and the whole antibody was expressed in combination. The prepared recombinant antibody expression plasmids were then transfected into HEK293 cells (such as HEK293F, Invitrogen Inc.) using liposomes (such as 293fectin, Invitrogen, Inc., etc) or other transfection reagents (such as PEI, etc). The cells were cultured in suspension in serum-free mediums for 3-5 days. The culture supernatant was then harvested by centrifugation. The recombinant antibody in the supernatant was subjected to one-step purification using Protein A/G affinity chromatography column (such as HisTrap FF, GE Inc, etc). The recombinant protein preservation buffer was then replaced with PBS (pH 7.0) or other suitable buffers using a desalting column (such as Hitrap desaulting, GE Inc., etc). If necessary, the sample can be sterilized by filtration and then stored in aliquots at -20°C.

### Example 2: Screening of the mouse monoclonal antibodies against the varicella-zoster virus recombinant protein gH/gL

### 2.1 Mouse immunization and preparation of immune antibody library

Recombinant protein gH-His/gL prepared in Example 1 was used as an antigen to immunize 6-8 weeks old of BALB/c mice at a dose of 50 µg/mouse, with booster immunization every 14 days. The mice were sacrificed 8 weeks after the initial immunization, and splenocytes were collected. Mouse spleen lymphocytes were isolated using a mouse lymphocyte isolation solution (Dakewe Biotech Co., Ltd., CAT#DKW33-R0100). Total RNA was extracted from the isolated lymphocytes using a cellular total RNA extraction kit (Tiangen Biotech (Beijing) Co., Ltd., CAT#DP430). Using the extracted total RNA as a template, cDNAs of the heavy chain variable region and light chain variable region of the antibody were synthesized using a first strand cDNA synthesis kit (Thermo scientific, CAT#K1621), respectively. Gene-specific primers were used as reverse transcription primers. The primer pairing regions were located in the heavy chain constant region and light chain constant region of the antibody, respectively, and the specific sequences were PmCGR: TGCATTTGAACTCCTTGCC (SEQ ID NO:23) and PmCKR: CCATCAATCTTCCACTTGAC (SEQ ID NO:24), respectively. The synthesized cDNAs were immediately stored at-70°C for later use. Then, using the cDNA obtained by reverse transcription as a template, the primers were synthesized by referring to the reference (Krebber A, Bornhauser S, Burmester J, et al., Reliable cloning of functional antibody variable domains from hybridomas and spleen cell repertoires employing a reengineered phage display system. J Immunol Methods. 1997; 201(1):35-55, which is incorporated by reference in its entirety). The nucleotide sequences encoding VH and VK of the mouse antibodies were amplified by PCR, respectively. Then, the single chain fragment variable (scFv) was constructed by overlapping extension PCR technology. Finally, the prepared nucleotide sequences encoding the mouse single chain fragment variable were cloned into the vector pADSCFV-S (see Example 1 of the Chinese Patent Application No. 201510097117.0 for detailed experimental protocols, which is incorporated by reference in its entirety) to construct a scFv library. The antibody library has a capacity of 2.13E+08, and an accuracy of 50%.

### 2.2 Screening of the mouse single chain fragment variables against the varicella-zoster virus recombinant protein gH/gL

Referring to the reference (see the Chinese Patent Application No. 201510097117.0 for detailed experimental protocols, which is incorporated by reference in its entirety), the above constructed phage library displaying the mouse single chain fragment variable was screened by a solid phase screening strategy (as for the experimental protocol, see, Phage Display: A Practical Approach, Clackson, T. (USA) and Lowman, H. B. (USA) (ed.), translated by Lan Ma et al., Chemical Industry Press, May 2008, which is incorporated by reference in its entirety) using the recombinant protein gH-His/gL prepared in Example 1 as an antigen. Three rounds of screening were carried out by means of binding, elution, neutralization, infection and amplification. Finally, a number of single chain fragment variables were obtained, which have different sequences, but can specifically bind to the recombinant protein gH-His/gL, including clones S7A10 (the amino acid sequence of which is set forth in SEQ ID NO:25), S8E1 (the amino acid sequence of which is set forth in SEQ ID NO:26) and S8B8 (the amino acid sequence of which is set forth in SEQ ID NO:27).

### Example 3: Affinity analysis of the mouse monoclonal antibodies against the varicella-zoster virus recombinant protein gH/gL

The nucleotide sequences encoding the light and heavy chains of the single chain fragment variable that specifically bind to gH/gL were cloned into a eukaryotic expression vector using conventional molecular biological methods so as to prepare a recombinant human IgG1-κ version of the mouse-human chimeric antibody.

The affinity of the anti-gH/gL antibody was determined by the surface plasmon resonance technique using Biacore X100. Reagents and consumables such as an amino-coupling kit (BR-1000-50), a human antibody capture kit (BR-1008-39), a CM5 chip (BR100012) and 10×HBS-EP (pH 7.4, BR100669) were purchased from GE healthcare. The surface of the carboxylated CM5 chip was activated with 1-ethyl-3-(3-dimethylaminopropyl) carbodiimide hydrochlorid (EDC) and N-Hydroxysuccinimide (NHS) according to the instructions in the kit. An anti-human IgG (Fc) antibody (capture antibody) was diluted to 25µg/mL with 10mM sodium acetate (pH5.0), and then injected at a flow rate of 10µL/min to achieve a coupling amount of approximately up to 10,000 response units (RU). After injection of the capture antibody, 1 M ethanolamine was injected to block unreacted groups. For the kinetics measurement, the anti-gH/gL antibody was diluted to 0.5-1µg/mL, and injected at 10µL/min to ensure that approximately 50RU of the antibody was captured by the anti-human Fc antibody. Recombinant protein gH/gL was then set to a series of concentration gradients (e.g., 1.23nM, 3.7nM, 11.1nM, 33.3nM and 100nM) and injected at 25°C from lower to higher concentrations at 30µL/min, with an association time of 120s and a dissociation time of 3,600s. A 3M MgCl₂ solution was injected at 10µL/min for 30s to regenerate the surface of the chip. The association rate (Kₐ) and dissociation rate (K_{d}) were calculated by fitting the binding and dissociation sensing grams with a 1:1 binding model using Biacore X100 evaluation software version 2.0.1. The dissociation equilibrium constant (K_{D}) was calculated as the ratio Kd/Ka. The fitting results were shown in Table 1.

**Table 1. Affinity constants for the binding of the recombinant monoclonal antibodies against the varicella-zoster virus recombinant protein gH/gL to the varicella-zoster virus recombinant protein gH/gL**

| | Kₐ | K_{d} | K_{D} |
|---|---|---|---|
| S8B8-IgG1 | 4.488E+5 | 2.068E-4 | 4.609E-10 |
| S7A10-IgG1 | 1.721E+5 | 9.531E-4 | 5.539E-9 |
| S8E1-IgG1 | 6.695E+4 | 2.995E-4 | 4.473E-9 |

### Example 4: Epitope analysis of the mouse monoclonal antibodies against the varicella-zoster virus recombinant protein gH/gL

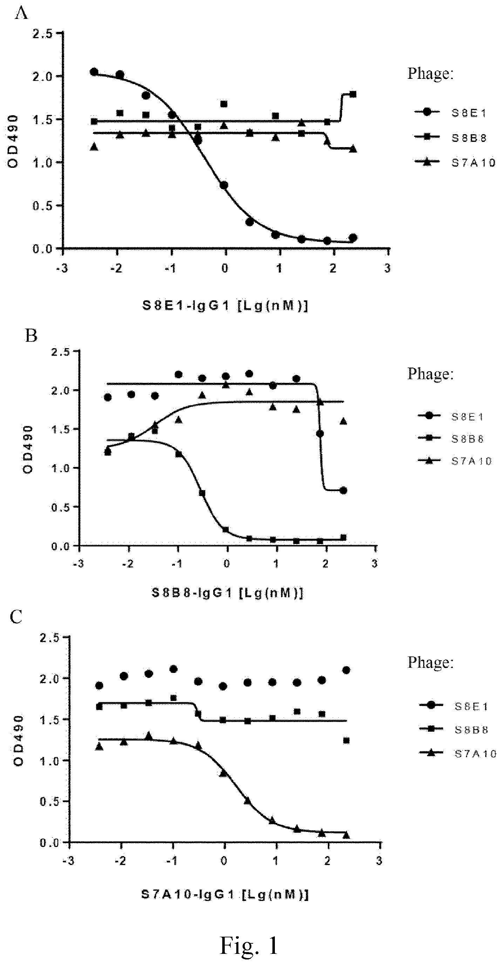

96-well ELISA plates were coated with the varicella-zoster virus recombinant protein gH/gL (3µg/mL, 100µL/well) overnight in a refrigerator at 4°C, and were then blocked with a blocking solution (PBS-0.1% Tween 20-3% milk) at 37°C for 1 hour. Gradient dilutions of anti-recombinant protein gH/gL antibodies (i.e. S7A10, S8B8 and S8E1) were performed with each of the purified anti-recombinant protein gH/gL phages (i.e., S7A10, S8B8 and S8E1) at a fixed concentration (5×10¹⁰-1×10¹¹cfu/mL), respectively. The starting concentration of the antibodies was 10µg/mL, and the dilution was carried out at 3-fold for a total of 8-10 concentration gradients. The antibody dilutions were added to the blocked 96-well ELISA plates at 100 µL/well and the plates were incubated at 37°C for 1 hour. Then, the ELISA plates were washed with PBS-0.1% Tween 20, followed by the addition of HRP-anti-M13 secondary antibody (Sino Biological Inc., 11973-MM05T-H) and the plates were incubated at 37°C for 1 hour. The ELISA plates were washed with PBS-0.1% Tween 20, and an OPD substrate chromogenic solution was added. After incubation for 5-10 minutes, the color development was terminated with 1M H₂SO₄. The optical density values were measured using a microplate reader at 492nm/630nm dual wavelength. The results of ELISA assay were shown in Figure 1. The monoclonal antibody S8E1-IgG1 could not block the binding of phages S8B8 and S7A10 to the recombinant protein gH/gL (Figure 1A). The monoclonal antibody S8B8-IgG1 could partially block the binding signal of the phage S8E1 to the recombinant protein gH/gL, but had no effect on the binding of the phage S7A10 to the recombinant protein gH/gL (Figure 1B). The monoclonal antibody S7A10-IgG1 could not block the binding of the phages S8B8 and S7A10 to the recombinant protein gH/gL (Figure 1C).

### Example 5: Neutralization activity of the mouse monoclonal antibodies against the varicella-zoster virus recombinant protein gH/gL

Neutralization activity of the antibodies (S7A10, S8B8 and S8E1) was determined by a reduction of plaque formation in MRCS cells infected with a mixture of an antibody and VZV MRC-5 cells were seeded in 6-well cell culture plates at a density of 1.5×10⁵ cells/well and cultured for 72 hours to form monolayer cells. At 90% confluence, the culture supernatant was removed and a virus maintenance solution (i.e., MEM medium containing 2% serum) was added at 400µL per well. VZV (Oka) was diluted to 2,000pfu/mL with the virus maintenance solution. That is, after the virus maintenance solution was mixed with the antibody in equal volume, every 100µL mixed solution comprised 100 pfu VZV The antibody was diluted by a 2-fold gradient with a viral dilution. The starting concentration of the antibodies was 20µg/mL, and there were 8 concentration gradients. That is, after mixing with an equal volume of VZV, the concentrations of the antibody were 10µg/mL, 5µg/mL, 2.5µg/mL, 1.25µg/mL, 0.62µg/mL, 0.31µg/mL, 0.16µg/mL and 0.08µg/mL. VZV was mixed with the antibody in equal volume and left to stand at 25°C for 1 hour to infect MRCS cells in 6-well plates (100µL/well). After adsorption at 37°C for 1 hour, the supernatant was pipetted and discarded. The plate was washed twice with PBS. 3mL of maintenance solution was added to each well, and the culture was maintained and cultured at 37°C for 8 days. Then the supernatant was discarded. The plates were washed once with PBS. 1 mL of Coomassie Brilliant Blue staining solution was added to each well. After 10 minutes, the plates were washed once with PBS, and were placed in a bright place to count plaque. The concentration of the antibody for 50% reduction in plaque (PRNT50) was calculated by Reed and Muench method. The experimental results were shown in Table 2.

**Table 2. Neutralization activity of the mouse monoclonal antibodies against the varicella-zoster virus recombinant protein gH/gL**

| Antibody | S7A10 | S8B8 | S8E1 |
|---|---|---|---|
| PRNT50 (µg/mL) | 0.34 | 0.38 | 0.38 |

### Example 6: Activity of the monoclonal antibodies against the varicella-zoster virus recombinant protein gH/gL in inhibiting virus transmission between cells

Antibodies (S7A10, S8B8 and S8E1) were added 24 hours after MRCS cells were infected by VZV The activity of the antibodies in inhibiting virus transmission between cells was determined by a reduction of plaque formation. MRC-5 cells were seeded in 6-well cell culture plates at a density of 1.5×10⁵ cells/well, and cultured for 72 hours to form monolayer cells. At 90% confluence, the culture supernatant was removed and a virus maintenance solution (i.e., MEM medium containing 2% serum) was added to each well at 400µL. VZV was diluted to 1,000pfu/mL with the virus maintenance solution to infect MRCS cells in 6-well cell culture plates (100µL/well). That is, cells in each well were infected with 100pfu of VZV After adsorption at 37°C for 1 hour, the supernatant was pipetted and discarded. The plates were washed twice with PBS. 3mL of maintenance solution was added to each well, and the culture was maintained and cultured at 37°C for 24 hours. The antibody was diluted by a 2-fold gradient with a viral dilution. The starting concentration of the antibody was 400µg/mL, and there were 8 concentration gradients. The antibody was added to MRCS cells infected with VZV in 6 well plates (30µL/well). That is, the concentrations of the antibody in the culture solutions were 4µg/mL, 2µg/mL, 1µg/mL, 0.5 pg/mL, 0.25 µg/mL and 0.125 µg/mL. The culture was maintained and cultured at 37°C for 8 days. The supernatant was discarded. The plates were washed once with PBS. 1 mL of Coomassie Brilliant Blue staining solution was added to each well. After 10 minutes, the plates were washed once with PBS, and were placed in a bright place to count plaque. The concentration of the antibody for 50% reduction in plaque was calculated. The experimental results were shown in Table 3.

**Table 3. Activity of the mouse monoclonal antibodies against the varicella-zoster virus recombinant protein gH/gL in inhibiting virus transmission between cells**

| Antibody | S7A10 | S8B8 | S8E1 |
|---|---|---|---|
| PRNT50(µg/mL) | 0.93 | 3.21 | 2.82 |

### Example 7: Screening of the fully human monoclonal antibody against the varicella-zoster virus recombinant protein gH/gL

A natural human phage antibody library was screened by a solid phase screening strategy (as for the experimental protocol, see, Phage Display: A Practical Approach, Clackson, T. (USA) and Lowman, H. B. (USA) (ed.), translated by Lan Ma et al., Chemical Industry Press, May 2008, which is incorporated by reference in its entirety) using the recombinant protein gH-His/gL prepared in Example 1 as an antigen (see Example 1 in the Chinese Patent Application No. 201510097117.0 for detailed experimental protocols, which is incorporated by reference in its entirety). Finally, a fully human single chain fragment variable H4H8-L1B7 was obtained, which specifically binds to the recombinant protein gH-His/gL.

### Example 8: Epitope analysis of the fully human monoclonal antibody against the varicella-zoster virus recombinant protein gH/gL

The nucleotide sequences encoding the light and heavy chains of H4H8-L1B7 were cloned into a eukaryotic expression vector using conventional molecular biological methods so as to prepare a recombinant human IgG1-κ version of the fully human monoclonal antibody.

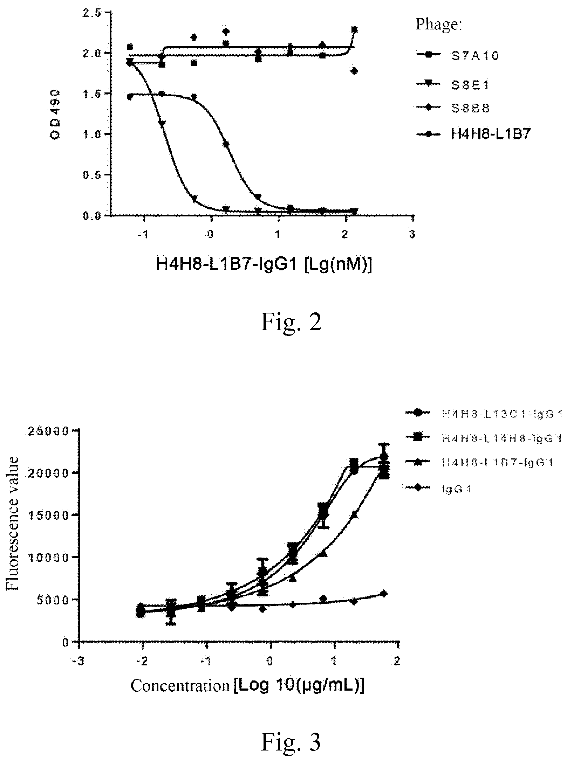

Referring to Example 4, gradient dilution of the anti-recombinant protein gH/gL antibody (*i.e.,* H4H8-L1B7-IgG1) was performed with each of the purified anti-recombinant protein gH/gL phages (*i.e.,* S7A10, S8E1, S8B8 and H4H8-L1B7) at a fixed concentration (5×10¹⁰-1×10¹¹cfu/mL), respectively. The blockage of the binding of the anti-recombinant protein gH/gL phages to the recombinant protein gH/gL by the human monoclonal antibody H4H8-L1B7-IgG1 against the recombinant protein gH/gLwas determined. The results of the ELISA assay were shown in FIG. 2. The monoclonal antibody H4H8-L1B7-IgG1 could completely block the binding signals of the phages H4H8-L1B7 and S8E1 to the recombinant protein gH/gL, and has no effect on the binding signals of the other two phages (S7A10 and S8B8) to the recombinant protein gH/gL.

### Example 9: Construction and screening of the light chain mutation library of the fully human monoclonal antibody H4H8-L1B7 against the varicella-zoster virus recombinant protein gH/gL

### 9.1 Construction and screening of H4H8-L1B7 light chain mutation library

The *in vitro* affinity maturation of the H4H8-L1B7 monoclonal antibody was performed using a light chain CDR mutation strategy based on a dual-vector phage display system (see Example 5 in Chinese Patent No. 201510097117.0 previously submitted by the applicant for the specific manipulation, which is incorporated by reference in its entirety). An LCDR3 mutation library with a library capacity of more than 5.5E+07 was constructed using the classical overlap extension PCR method. The design scheme of the mutation library was shown in Table 4.

**Table 4. The design scheme of the LCDR3 mutation library**

| Initial amino acid | | Mutant amino acid | Degenerate code | |
|---|---|---|---|---|
| | Q | Q, E, L, V, R or G | | SDG |
| | Q | Q, E, L, V, R or G | | SDG |
| | A | G, V, A, D, I, T, N or S | | RNT |
| | N | F, I, S, T, Y or N | | WHT |
| | N | F, I, S, T, Y or N | | WHT |
| | F | F, I, S, T, Y or N | | WHT |
| | P | F, S, L, P, A or V | | BYC |
| | L | L, S, I, T, A or V | | DYC |
| | T | S, T, A, Y, N or D | | DMT |

The LCDR3 mutation library of L1B7 (SEQ ID NO:28) was subjected to 2 rounds of screening by a solid-phase screening strategy using the recombinant protein gH-His/gL as an antigen and on the basis of the heavy chain of H4H8-L1B7. Finally, three light chain mutants, L13C1(SEQ ID NO:8), L13C7(SEQ ID NO:9) and L14H8(SEQ ID NO: 10) were obtained.

### 9.2 Affinity analysis of the light chain mutants of H4H8-L1B7

The nucleotide sequences encoding the light and heavy chains of H4H8-L14H8, H4H8-L13C1 and H4H8-L13C7 were cloned into eukaryotic expression vectors using conventional molecular biological methods so as to prepare the recombinant human IgG1-κ versions of the fully human monoclonal antibodies.

Referring to Example 3, the affinity of the light chain mutants of H4H8-L1B7 was analyzed using Biacore X100 evaluation software version 2.0.1. The results were shown in Table 5.

**Table 5. Affinity constants for the binding of the light chain mutants of H4H8-L1B7 to the recombinant protein gH/gL**

| | Kₐ | K_{d} | K_{D} |
|---|---|---|---|
| H4H8-L13C1-IgG1 | 2.601E+5 | 1.281E-4 | 4.925E-10 |
| H4H8-L13C7-IgG1 | 2.494E+5 | 1.211E-4 | 4.855E-10 |
| H4H8-L14H8-IgG1 | 3.666E+5 | 1.062E-4 | 2.896E-10 |

### Example 10: Neutralization activity of the monoclonal antibodies against the varicella-zoster virus recombinant protein gH/gL

Referring to Example 5, the neutralization activity of the monoclonal antibodies H4H8-L14H8, H4H8-L13C1 and H4H8-L13C7 was determined. The experimental results were shown in Table 6.

**Table 6. Neutralization activity of the monoclonal antibodies against the varicella-zoster virus recombinant protein gH/gL**

| | H4H8-L14H8 | H4H8-L13C1 | H4H8-L13C7 |
|---|---|---|---|
| PRNT50(µg/mL) | 0.016 | 0.016 | >0.4 |

### Example 11: Activity of the monoclonal antibodies against the varicella-zoster virus recombinant protein gH/gL in inhibiting virus transmission between cells

Referring to Example 6, the activity of the monoclonal antibodies H4H8-L14H8, H4H8-L13C1 and H4H8-L13C7 in inhibiting virus transmission between cells was determined. The experimental results were shown in Table 7.

**Table 7. Activity of the monoclonal antibodies against the varicella-zoster virus recombinant protein gH/gL in inhibiting virus transmission between cells**

| | H4H8-L14H8 | H4H8-L13C1 | H4H8-L13C7 |
|---|---|---|---|
| PRNT50(µg/mL) | 0.31 | 0.28 | >2 |

### Example 12: Anti-varicella-zoster virus monoclonal antibodies mediate ADCC effects on the infected cells

The present inventors constructed a Jurkat-Dual-CD16a reporter gene cell strain by transforming CD16a plasmid into Jurkat-dual cells purchased from Invivogen Inc. When an antibody-dependent cell-mediated cytotoxicity (ADCC) effect occurrs, the activation signal was transferred to the downstream NF-κB pathway through the CD16a molecule. ADCC activity was finally detected by luciferase. 90% confluent MRCS cells in a T75 cell culture flask were infected by varicella-zoster virus (VZV) at MOI=0.01. The cells were observed under a microscope. When 50%-60% of the cells in the flask were diseased (96 hours after infection), the cells were subjected to trypsinization and harvested. Jurkat-Dual-CD16a cells and target cells infected with VZV (MRC-5 cells) were mixed in the effector-to-target ratio of 15:1. The dilutions of the molecules to be tested H4H8-L14H8-IgG1, H4H8-L13C1-IgG1, H4H8-L1B7-IgG1 and the isotype control IgG1 antibody were all started at a final concentration of 60µg/mL, with a 3-fold dilution for a total of 10 concentration points. The diluted antibodies were co-cultured with the above mixed cells, respectively. After incubation for 20 h, the mixture was centrifuged and 50µL supernatant was taken. An automatic loading program was set in a microplate reader. 50µL luciferase detection solution quanti-luc (Invivogen Inc., Cat. No. rep-qlc2) was added to each well and the fluorescence intensity was measured by a full wavelength detection. The results of ADCC activity based on Jurkat-Dual-CD16a reporter gene system showed that H4H8-L14H8-IgG1 and H4H8-L13C1-IgG1 had better ADCC activity than H4H8-L1B7-IgG1 (FIG. 3).

Exemplary embodiments of the inventions of the present application have been described above. However, those skilled in the art can modify or improve the exemplary embodiments described herein without departing from the spirit and scope of the present application, and variations or equivalents derived therefrom also fall within the scope of the present application.

### Reference

Davison, A. J., and J. E. Scott. 1986. The complete DNA sequence of varicella-zoster virus. J. Gen. Virol. 67:1759-1816.
Davison, A. J., C. M. Edson, R. W. Ellis, B. Forghani, D. Gilden, C. Grose, P. M. Keller, A. Vafai, Z. Wroblewska, and K. Yamanishi. 1986. A new common nomenclature for the glycoprotein genes of varicella-zoster virus and their glycosylated products. J. Virol. 57:1195-1197.
Kapsenberk, J. G. 1964. Possible antigenic relationship between varicellazoster virus and herpes simplex virus. Arch. Ges. Virusforsch. 15:67-73.
Forghani, B., L. Ni, and C. Grose. 1994. Neutralization epitope of the varicella-zoster virus gH:gL glycoprotein complex. Virology 199:458-462.
Zhu, Z., M. D. Gershon, R. Ambron, C. Gabel, and A. A. Gershon. 1995. Infection of cells by varicella-zoster virus: inhibition of viral entry by mannose 6-phosphate and heparin. Proc. Natl. Acad. Sci. USA 92:3546-3550.
Sadzot-Delvaux, C., M. P. Merville-Louis, P. Delree, P. Marc, J. Piette, G. Moonen, and B. Rentier. 1990. An in vivo model of varicella-zoster virus latent infection of dorsal root ganglia. J. Neurosci. Res. 26:83-89.
Gershon, A. A., S. P. Steinberg, and L. Gelb. 1984. Clinical reinfection with varicella-zoster virus. J. Infect. Dis. 149:137-142.
Guess, H. A., D. D. Broughton, L. J. Melton, and L. T. Kurland. 1985. Epidemiology of herpes zoster in children and adolescents: a populationbased study. Pediatrics 76:512-518.
Bogger-Goren, S., J. M. Bernstein, A. A. Gershon, and P. L. Ogra. 1984. Mucosal cell-mediated immunity to varicella-zoster virus: role in protection against disease. J. Pediatr. 105:195-199
Zaia, J. A., M. J. Levin, S. R. Preblud, J. Leszczynski, G. G. Wright, R. J. Ellis, A. C. Curtis, M. A. Valerio, and J. LeGore. 1983. Evaluation of varicella-zoster immune globulin: protection of immunosuppressed children after household exposure to varicella. J. Infect. Dis. 147:737-743.
Gershon, A.A.; Steinberg, S.; Gelb, L.; NIAID-Collaborative-Varicella-Vaccine-Study-Group. Live attenuated varicella vaccine: Efficacy for children with leukemia in remission. JAMA 1984, 252, 355-362.
Chen, J.; Gershon, A.; Silverstein, S.J.; Li, Z.S.; Lungu, O.; Gershon, M.D. Latent and lytic infection of isolated guinea pig enteric and dorsal root ganglia by varicella-zoster virus. J. Med. Virol. 2003, 70, S71-S78.
Oxman, M.N.; Levin, M.J.; Johnson, G.R.; Schmader, K.E.; Straus, S.E.; Gelb, L.D.; Arbeit, R.D.; Simberkoff, M.; Gershon, A.; Davis, L.E.; et al. A vaccine to prevent herpes zoster and postherpetic neuralgia in older adults. N. Engl. J. Med. 2005, 352, 2271-2284.
Cunningham, A.L.; Lal, H.; Kovac, M.; Chlibek, R.; Hwang, S.J.; Diez-Domingo, J.; Godeaux, O.; Levin, M.; McElhaney, J.E.; Puig-Barbera, J.; et al. Efficacy of the Herpes Zoster Subunit Vaccine in Adults 70 Years of Age or Older. N. Engl. J. Med. 2016, 375, 1019-1032.

## Claims

1. An antibody against varicella-zoster virus comprising a heavy chain variable region comprising the amino acid sequences of HCDR1, HCDR2 and HCDR3 and a light chain variable region comprising the amino acid sequences of LCDR1, LCDR2 and LCDR3, wherein
the amino acid sequence of HCDR1 is SGNYWN, the amino acid sequence of HCDR2 is YISYDGSTYYNPSLKN, the amino acid sequence of HCDR3 is GYYGYWFAY, the amino acid sequence of LCDR1 is RASSSVSYMH, the amino acid sequence of LCDR2 is ATSNLAS, and the amino acid sequence of LCDR3 is QQWSSNPFT; or
the amino acid sequence of HCDR1 is SGYYWN, the amino acid sequence of HCDR2 is YISYDGSNNYNTSLKN, the amino acid sequence of HCDR3 is EDVNYPPYALDY, the amino acid sequence of LCDR1 is RSSQSLVHSNGNTYLH, the amino acid sequence of LCDR2 is KVSNRFS, and the amino acid sequence of LCDR3 is SQSTHVPWT; or
the amino acid sequence of HCDR1 is SYTMS, the amino acid sequence of HCDR2 is FISNGGDNNYYADTVKG, the amino acid sequence of HCDR3 is HNGNWGFAY, the amino acid sequence of LCDR1 is SASSSISSNYLH, the amino acid sequence of LCDR2 is RTSNLAS, and the amino acid sequence of LCDR3 is QQGSSIPLT; or
the amino acid sequence of HCDR1 is TYAMH, the amino acid sequence of HCDR2 is VISYGGGNRYYAASVKG, the amino acid sequence of HCDR3 is ARDNHYFFGMDV, the amino acid sequence of LCDR1 is RASQGISSWLA, the amino acid sequence of LCDR2 is AASSLQS, and the amino acid sequence of LCDR3 is QQANSFPLT, QEGFYFPIN, QQATYFPIN or QQSSYFPIA;
wherein the amino acid sequences of HCDRs and LCDRs are defined according to Kabat.

2. The antibody according to Claim 1, wherein the amino acid sequence of the heavy chain variable region of the antibody is set forth in SEQ ID NO: 1, 3, 5, or 7.

3. The antibody according to Claim 1, wherein the amino acid sequence of the light chain variable region of the antibody is set forth in SEQ ID NO: 2, 4, 6, 8, 9, 10, or 28.

4. The antibody according to Claim 1, wherein
the amino acid sequence of the heavy chain variable region of the antibody is set forth in SEQ ID NO:1, and the amino acid sequence of the light chain variable region of the antibody is set forth in SEQ ID NO:2; or
the amino acid sequence of the heavy chain variable region of the antibody is set forth in SEQ ID NO:3, and the amino acid sequence of the light chain variable region of the antibody is set forth in SEQ ID NO:4; or
the amino acid sequence of the heavy chain variable region of the antibody is set forth in SEQ ID NO:5, and the amino acid sequence of the light chain variable region of the antibody is set forth in SEQ ID NO:6;
the amino acid sequence of the heavy chain variable region of the antibody is set forth in SEQ ID NO:7, and the amino acid sequence of the light chain variable region of the antibody is set forth in SEQ ID NO:8; or
the amino acid sequence of the heavy chain variable region of the antibody is set forth in SEQ ID NO:7, and the amino acid sequence of the light chain variable region of the antibody is set forth in SEQ ID NO:9; or
the amino acid sequence of the heavy chain variable region of the antibody is set forth in SEQ ID NO:7, and the amino acid sequence of the light chain variable region of the antibody is set forth in SEQ ID NO: 10; or
the amino acid sequence of the heavy chain variable region of the antibody is set forth in SEQ ID NO:7, and the amino acid sequence of the light chain variable region of the antibody is set forth in SEQ ID NO:28.

5. An antibody against varicella-zoster virus, wherein the amino acid sequence of the heavy chain variable region of the antibody has at least 90% identity to the amino acid sequence as set forth in any one of SEQ ID NOs: 1, 3, 5, and 7, and the amino acid sequence of the light chain variable region of the antibody has at least 90% identity to the amino acid sequence as set forth in any one of SEQ ID NOs: 2, 4, 6, 8, 9, 10, and 28.

6. The antibody according to any one of claims 1-5, wherein
the antibody is a whole antibody, a Fab fragment, a F(ab')₂ fragment, or a single chain Fv (scFv); preferably, the antibody is a fully human antibody; and/or
the antibody is a monoclonal antibody; and/or
the antibody further comprises a heavy chain constant region selected from an IgG1 subtype, an IgG2 subtype, or an IgG4 subtype; preferably, the heave chain constant region is an IgG1 subtype; and/or
the antibody further comprises a light chain constant region selected from a kappa subtype or a lamda subtype; preferably, the light chain constant region is a kappa subtype.

7. The antibody according to any one of claims 1-6, wherein
the antibody binds to the gH/gL protein of varicella-zoster virus; and/or
the antibody mediates antibody-dependent cell-mediated cytotoxicity (ADCC).

8. A nucleic acid molecule encoding the antibody according to any one of claims 1-7.

9. A pharmaceutical composition comprising the antibody according to any one of claims 1-7 and a pharmaceutically acceptable excipient, diluent or carrier.

10. The pharmaceutical composition according to claim 9, for use in the prevention or treatment of varicella and herpes zoster.

11. Use of the antibody according to any one of claims 1-7, the nucleic acid molecule according to claim 8, or the pharmaceutical composition according to claim 9 or 10 in the manufacture of a medicament for the prevention or treatment of varicella and herpes zoster.

12. A method of preventing or treating varicella and herpes zoster, comprising administering to a subject in need thereof the antibody according to any one of claims 1-7, or the pharmaceutical composition according to claim 9 or 10.
